# EUROPEAN PATENT SPECIFICATION

(11) **EP 0 778 023 B1**
(45) Date of publication and mention of the grant of the patent: **12.03.2003**
(21) Application number: 96308786.1
(22) Date of filing: 04.12.1996
(51) Int. Cl.: A61K 31/445, A61P 25/08, A61P 25/14

(54) **Product comprising rapamycin or particular derivatives and an NMDA or AMPA antagonist for use for the treatment of epilepsy or Huntington's Disease, and the use of rapamycin or particular derivatives for the manufacture of a medicament for the treatment of epilepsy or Huntington's Disease.**
Produkt enthaltend Rapamycin oder ausgewählte Derivate und einen NMDA- oder AMPA-Antagonisten zur Verwendung für die Behandlung von Epilepsie oder Morbus Huntington, und die Verwendung von Rapamycin oder von ausgewählten Derivaten für die Herstellung eines Medikaments für die Behandlung von Epilepsie oder Morbus Huntington.
Produit contentant de la rapamycin ou des derivés selectionnés et un NMDA- ou AMPA-antagoniste utilisé pour le traitement de l'épilepsie ou de la maladie d'Huntington, et l'utilisation de la rapamyin ou des derivés selectionnés pour la production d'un médicament pour le traitement de l'épilepsie ou de la maladie d'Huntington.

(30) Priority: 07.12.1995 US 8337
(43) Date of publication of application: 11.06.1997
(73) Proprietor: Wyeth, Madison, New Jersey 07940-0874 (US)
(72) Inventor: Lin, Stephen Shi-hsun, Princeton, New Jersey 08540 (US); Molnar-Kimber, Katherine Lu, Glenside, Pennsylvania 19038 (US)
(74) Representative: Wileman, David Francis, Dr.

(56) References cited:
- EP-A- 0 467 606
- EP-A- 0 507 555
- EP-A- 0 532 862
- EP-A- 0 650 730
- WO-A-92/14477
- DE-A- 4 118 740
- US-A- 5 206 018
- US-A- 5 260 299

## Description

This invention relates to neuroprotective agents, more particularly to a new use of rapamycin and certain derivatives thereof.

Glutamate induced toxicity is hypothesized to be the mechanism of neuronal cell death in ischemia, stroke, and head injury. [Simon RP, Science 226:850 (1984); Benveniste H, J. Neurochem. 43:1369 (1984)]. It is also implicated in other neurological disorders such as Alzheimer's, ALS, Epilepsy, Huntington's and Parkinson's disease. [Beal F, Nature 321: 168 (1986); Maragos WF, Trends Neurosci. 10:65 (1987); Greenamyre JT, Neurobiol. Aging 10:593 (1989); Koh J-Y, Brain Res. 533:315 (1990); Mattson MP, J. Neurosci. 12:376 (1992); Rothstein, JD, New England J. Med. 326:1464 (1992); Choi, DW, New England J. Med. 326(22): 1493-4 (1992)]. Under normal conditions glutamate functions as an excitatory neurotransmitter in the brain. It is released from presynaptic terminals and activates receptors on postsynaptic neurons. The activated receptors allow sodium and calcium ions to flow into the cell to produce an excitatory response. The action of these excitatory amino acids is mediated by several distinct receptor subtypes of which the best studied one is the N-methyl-D-aspartate (NMDA) receptor. Excessive activation of the NMDA receptor complex may cause neuronal overstimulation with pathological consequences. Under pathological conditions, excessive levels of glutamate accumulate in the extracellular space leading to an overstimulation of glutamate receptors. This induces a greater influx of sodium and calcium, resulting in high levels of intracellular calcium which initiate as yet unknown processes of cell death.

Antagonists of glutamate receptors are currently being developed to prevent neuronal cell death following stroke or head trauma. [Hirose K, Neurochem. Res. 18:479 (1993); Dugan LL, Annals of Neurology, Vol. 35 suppl. S17-19 (1994)] A number of studies have demonstrated that a blockade of the NMDA-subclass receptor significantly reduces a neuronal damage and loss which occurs in animal models mimicking a variety of neuropathological situations. These observations strongly indicate that NMDA antagonists offer neuroprotection in several clinical settings. However, many of these drugs are effective only when given within 6 hours following the insult or injury. This may be due to the fact that intracellular calcium levels can also rise due to calcium entering the cell through voltage gated calcium channels. Furthermore, since activation of glutamate receptors is required for normal synaptic transmission and brain activity, prolonged treatments with glutamate receptor blockers is not feasible. This limits their use as neuroprotective agents in chronic neurodegenerative disorders. Therefore, drugs that can prevent neuronal cell death following the rise in intracellular calcium are appropriate for the delayed treatment of stroke and head injuries, as well as for chronic treatment of neurodegenerative diseases.

Several hypotheses have been proposed to explain how high levels of intracellular calcium lead to cell death. One is that it activates calcium dependent proteases such as calpain. Another is that it activates calcineurin, a calcium/calmodulin dependent phosphatase that dephoshorylates nitric oxide synthase (NOS). Dephosphorylation of NOS enables it to produce nitric oxide, which is toxic to cells. A third hypothesis is that high intracellular calcium levels induce apoptosis, or programmed cell death. These various hypotheses are not mutually exclusive and it is likely that all of these events, as well as others not yet discovered, are triggered by high levels of intracellular calcium to produce cell death.

In apoptosis, some genes that are needed for cell division are activated as if the cell is trying to divide. [Heintz N, Trends-Biochem-Sci. 18: 157 (1993)]. Many highly differentiated cells, such as neurons, have lost the capability to divide and in failing to do so, they die. Therefore, it is possible that drugs that can inhibit cells from dividing may also prevent them from undergoing apoptosis.

This invention provides use of rapamycin, rapamycin 1,3-Diels Alder adduct with phenyltriazolinedione, rapamycin 42-ester with 4-[[4-(dimethylamino)phenyl]azo]-benzenesulfonic acid, rapamycin 1,3-Diels Alder adduct with methyltriazolinedione, or rapamycin-O-benzyl-27-oxime for the manufacture of a medicament for treating epilepsy or Huntington's Disease in a mammal.

This invention also provides use of a compound selected from rapamycin, rapamycin 1,3-Diels Alder adduct with phenyltriazolinedione, rapamycin 42-ester with 4-[[4-(dimethylamino)phenyl]azo]-benzenesulfonic acid, rapamycin 1,3-Diels Alder adduct with methyltriazolinedione, and rapamycin-O-benzyl-27-oxime (collectively referred to as the compounds of this invention) for the manufacture of a medicament for treating epilepsy or Huntington's Disease in a mammal in which the medicament is adapted for administration orally, parenterally, intravascularly, intranasally, intrabronchially, transdermally, or rectally.

The compounds of this invention are also useful in inhibiting neuronal cell death related to the above disease states, when used in combination with an NMDA and/or AMPA antagonist.

When the compounds of this invention are used in combination with an NMDA and/or AMPA antagonist, the combination can be administered simultaneously or sequentially without regard to the order of administration.

Preferred NMDA antagonists include [2-(8,9-Dioxo-2,6-diazabicyclo[5.2.0]-non-1(7)-en-2-yl)ethyl]phosphonic acid (disclosed in U.S. Patent 5,168,103), D-amino-5-phosphonopentanoate (D-AP5), 4-phosphonomethyl-2-piperidinecarboxylic acid (CGS19755), D, L-(E)-2-amino-4-methylphosphono-3-pentanoic acid (CGP37849), 5,7-dichlorokynurenate, trans-2-carboxy-5,7-dichloro-4-phenylaminocarbonylamino-1,2,3,4-tetrahydroquinoline (L689560), and 5, 7-dinitroquinoxoline-2, 3-dione (MNQX); and preferred AMPA antagonists include 6-(lH-Imidazol-1-yl)-7-nitro-2,3(1H,4H)-quinoxalinedione hydrochloride (YM90K) (J. Med. Chem. 37: 647 (1994), 1-(4-aminophenyl)4-methyl-7,8-methylenedioxy-5H-2,3-benzodiazepine HCl (GYKI52466), and 6-nitro-7-sulphamobenzo(f)quinoxaline-2,3-dione (NBQX), 6-cyano-7-nitroquinoxaline-2,3-dione (CNQX), and 6,7-dinitroquinoxaline-2,3-dione (DNQX).

Treating covers treatment of an existing condition, inhibiting the progress or development of the condition, ameliorating the condition, and providing palliation of the condition.

Accordingly this invention provides a product containing rapamycin, rapamycin 1,3-Diels Alder adduct with phenyltriazolinedione, rapamycin 42-ester with 4-[[4-(dimethylamino)phenyl]azo]-benzenesulfonic acid, rapamycin 1,3-Diels Alder adduct with methyltriazolinedione, or rapamycin-O-benzyl-27-oxime and a NMDA or AMPA antagonist as a combined preparation for simultaneous, separate or sequential use in treating epilepsy or Huntington's Disease in a mammal. In a further respect this invention provides a pharmaceutical composition comprising rapamycin and a NMDA or AMPA antagonist and a pharmaceutically acceptable carrier.

The ability of the compounds of this invention to act as neuroprotective agents was demonstrated in the following in vitro standard pharmacological test procedures that emulate mammalian neurotoxicity.

The first standard pharmacological test procedure demonstrated the ability of the compounds of this invention to inhibit neuronal cell death resulting from glutamate induced toxicity. Briefly, rat hippocampal and cortical cultures were prepared according to the method of Furshpan and Potter with some modifications. [Neuron 3:199 (1989)]. Brains were removed from newborn Long Evans pups anesthetized with an injection of 0.1 ml of 20% chloral hydrate. The hippocampi were dissected out and separated from the rest of the cortex. Both tissues were enzymatically digested in 5 ml Earle's Balanced Salt Solution (EBSS) containing 20 u/ml papain, 1 mM L-cysteine, and 0.5 mM EDTA for 30 minutes at 37°C with gentle rocking. The hippocampi and cortical tissues were then washed once with EBSS and the digestion was stopped by incubating at 37°C for 30 minutes with 3-5 mis of inhibitor solution containing 1 mg/ml ovomucoid and 1 mg/ml albumin in EBSS.
The digested hippocampi and cortical tissues were washed twice with DME without L-glutamine with 4.5 g/L dextrose before being triturated in 2 ml of DME supplemented with 5% fetal calf serum, 5% rat serum, 50 u/ml penicillin, 0.05 mg/ml streptomycin and MlTO+ serum extender [a medium supplement from Collaborative Biomedical Products] (10% DME). After 50 triturations, undissociated tissue was allowed to settle and the dissociated cell suspension was removed. A fresh 2 ml of 10% DME was added to the remaining tissue and the trituration was repeated. The two cell suspension aliquots were pooled and the cell density was determined with a hemacytometer.

30,000 hippocampal cells in 150 µL were plated per well in 96-well plates and incubated at 37°C in 5% CO₂. Cortical cells were plated at a density of 500,000 cells per well in 24-well plates. When glia cells were confluent (after approximately 3 days), 4-10 µM cytosine β-D-arabinofuranoside was added to each well to prevent further proliferation. One week after plating, the medium was removed and replaced with DME supplemented with 2.5% fetal calf serum, 2.5% rat serum, 50 u/ml penicillin, 0.05 mg/ml streptomycin, MITO+, 1 mM kynurenate and 10 mM MgCl₂ (5% DME + KM). Kynurenate is a nonspecific blocker of glutamate receptors and elevated levels of magnesium block calcium and sodium influx through NMDA receptor channels. The addition of KM prevents neurotoxicity that occurs with glutamate found in serum, and allows neurons to be maintained in culture for up to several months. The cells were fed weekly by exchanging half the medium with fresh 5% DME + KM.

The compound to be evaluated was dissolved in DMSO to make 10 mM stock solutions and stored in aliquots at -80°C. Just before each procedure was carried out, aliquots of the stock solutions were thawed and diluted in DME or Hanks' Balanced Salt Solution (HBSS) containing 44 mM sodium bicarbonate, 10 mM glucose, and 30 µM glutamate to obtain test concentrations from 1 nM to 10 µM.

After 4-12 weeks in culture, hippocampal cells were washed three times and fed with test compound in HBSS, or serum free DME (SF DME) and 30 to 150 µM glutamate. At least three wells in every test procedure were treated with each drug concentration. 30 µM Glutamate kills 70-80% of the neurons in younger cultures, and more in older cultures. The toxicity induced by 30 µM glutamate was blocked by 1 mM kynurenate + 10 mM MgCl₂ (KM).

Following overnight incubation at 37°C, the number of live hippocampal neurons remaining in five fields, representing the north, south, east, and west extremes and the center of each well, were counted. Each field was visualized through a 10x objective with a 15x eyepiece and was approximately 1.54 mm² in area. Conditions were generally done in triplicate. The mean number of neurons per well and the standard deviation for each condition were calculated. The mean number of neurons remaining in the control 30 µM glutamate condition reflects those that were not sensitive to glutamate toxicity.

The following table shows the results obtained for the compound of Example 1.

| Compound | Concentration | No. Neurons Alive | S.D. |
|---|---|---|---|
| Example 1 | 10nM | 114 | 5 |
| Example 1 | 100nM | 92 | 38 |
| Example 1 | 1µM | 46 | 10 |
| Example 1 | 10µM | 47 | 20 |
| SF DME | | 28 | 4 |
| Kynurenate | 1 mM | 82 | 17 |

The following table shows the test results obtained for the compounds of this invention.

| Compound | Concentration | No. Neurons Alive | S.D. |
|---|---|---|---|
| Example 1 | 200 nM | 229 | 30 |
| Example 1 | 20 nM | 71 | 3 |
| Example 1 | 2 nM | 63 | 4 |
| Example 2 | 200 nM | 127 | 38 |
| Example 2 | 20 nM | 180 | 64 |
| Example 2 | 2 nM | 110 | 36 |
| Example 3 | 200 nM | 241 | 46 |
| Example 3 | 20 nM | 285 | 40 |
| Example 3 | 2 nM | 98 | 21 |
| Example 4 | 200 nM | 157 | 56 |
| Example 4 | 20 nM | 130 | 29 |
| Example 4 | 2 nM | 117 | 32 |
| Example 5 | 200 nM | 135 | 11 |
| Example 5 | 20 nM | 96 | 12 |
| Example 5 | 2 nM | 131 | 19 |
| Kynurenate | 1 mM | 202 | 40 |
| HBSS | | 53 | 25 |

The results of this standard pharmaceutical test procedure show that the compounds of this invention inhibit glutamate induced neurotoxicity and are therefore useful as a neuroprotective agent. As shown in the first table, lower concentrations of the compound of Example 1 (10 and 100 nM) proved superior to higher concentrations of the compound of Example 1 (1 and 10 µM), possibly due to some cytotoxic effects of the compound of Example 1 at these higher concentrations. Evaluation of the compounds of this invention in concentration ranges between 2 and 200 nM demonstrated neuroprotective activity against glutamate induced toxicity, that emulates mammalian neurotoxicity. At higher glutamate concentrations (75 µM), the compounds did not prevent neuronal cell death. The compounds of this invention were less effective as neuroprotective agents against glutamate induced toxicity in older hippocampal cultures (> 8 weeks), which are more sensitive to glutamate toxicity.

The second in vitro standard pharmacological test procedure demonstrates the ability of the compounds of the invention to augment the neuroprotective effects observed with NMDA and AMPA inhibitors. Briefly, cortical cultures were grown for eight weeks in 24-well plates as described above. Cortical neurons were washed six times with DME without glucose that has been deoxygenated by bubbling the medium with 95% N₂/5% CO₂ for at least 10 minutes, to induce an ischemic state that will cause neuronal cell death that can be observed by measuring lactate dehydrogenase levels, as the amount of LDH activity in each well is directly correlated with the number of dead cells. 100 µM [2-(8,9-Dioxo-2,6-diazabicyclo[5.2.0]non-1(7)-en-2-yl)-ethyl]-phosphonic acid (disclosed in U.S. Patent 5,168,103 and 10 µM YM90K were added to certain wells to block NMDA and AMPA glutamate receptors, respectively. To test wells, 1 µM of the compound of Example 1 was added. Control wells were washed with normal DME with glucose and 100 µM [2-(8,9-dioxo-2,6-diazabicyclo[5.2.0]non-1(7)-en-2-yl)ethyl]phosphonic acid. 5 µL samples were taken from each well at different time points following ischemia and the LDH activity in those samples was determined by adding 250 µL of 2 mg NADH/25 mL phosphate buffer to each sample. After incubating for 20-30 minutes, 10 µL of 22.7 mM pyruvate was added and the optical density (OD) at 340 nm read. The change in optical density with time (mOD/min) reflects the amount of LDH in the sample. Higher optical density changes means more LDH activity indicating more cell death. Samples were taken from 4-8 wells per condition and the average and standard deviation for each condition and time point determined. The results obtained are shown in the following table.

| LDH Activity (± S.D.) | | | | |
|---|---|---|---|---|
| Time (h) | Control | Ischemia | AMPA/NMDA | AMPA/NMDA/Example 1 |
| 0 | 0.31 ± 0.41 | 0.27 ± 0.05 | 0.69 ± 0.70 | 0.67 ± 0.29 |
| 0.5 | 0.51 ± 0.14 | 0.57 ± 0.29 | 0.45 ± 0.25 | 0.80 ± 0.28 |
| 1 | 0.40 ± 0.07 | 0.56 ± 0.16 | 0.46 ± 0.23 | 0.90 ± 0.37 |
| 1.5 | 0.43 ± 0.15 | 0.84 ± 0.44 | 0.38 ± 0.30 | 1.03 ± 0.32 |
| 4 | 0.64 ± 0.01 | 9.11 ± 1.63 | 2.29 ± 0.50 | 2.09 ± 0.58 |
| 5 | 0.68 ± 0.20 | 31.65 ± 5.76 | 16.07 ± 2.9 | 4.21 ± 1.02 |
| 6 | 0.78 ± 0.22 | 40.52 ± 6.43 | 29.99 ± 9.11 | 9.11 ± 7.04 |
| 7 | 1.28 ± 0.41 | 50.30 ± 6.96 | 44.56 ± 7.73 | 15.77 ± 4.68 |
| 8 | 1.15 ± 0.28 | 60.05 ± 8.66 | 61.77 ± 13.96 | 24.43 ± 7.87 |

The compounds of this invention were also evaluated in the standard test procedure emulating ischemic neurotoxicity, with the exception that 1 week old cortical cell cultures, that are less sensitive to ischemia mediated neurotoxicity, were used. Test compounds were administered with the NMDA and AMPA inhibitors as described above. Cyclosporin was also evaluated for the purpose of comparison.

| Time (hours) | LDH Activity | Std. Deviation |
|---|---|---|
| Control | | |
| 8 | 0.30 | 0.54 |
| 13 | 1.37 | 1.58 |
| 24 | 2.97 | 2.67 |
| 30 | 4.44 | 2.50 |

| Ischemia | | |
|---|---|---|
| 8 | 0.49 | 0.36 |
| 13 | 6.17 | 1.10 |
| 24 | 16.40 | 4.29 |
| 30 | 21.49 | 3.35 |

| Example 1 | | |
|---|---|---|
| 8 | 1.04 | 0.20 |
| 13 | 3.08 | 0.71 |
| 24 | 9.00 | 1.64 |
| 30 | 14.02 | 2.4 |

| Example 2 | | |
|---|---|---|
| 8 | 1.08 | 0.34 |
| 13 | 2.31 | 1.41 |
| 24 | 7.69 | 3.13 |
| 30 | 12.43 | 3.85 |

| Example 3 | | |
|---|---|---|
| 8 | 0.84 | 0.20 |
| 13 | 2.36 | 1.41 |
| 24 | 7.03 | 3.24 |
| 30 | 11.25 | 2.62 |

| Example 4 | | |
|---|---|---|
| 8 | 0.91 | 0.34 |
| 13 | 2.54 | 1.02 |
| 24 | 8.21 | 4.14 |
| 30 | 13.31 | 3.41 |

| Example 5 | | |
|---|---|---|
| 8 | 0.74 | 0.38 |
| 13 | 1.65 | 0.81 |
| 24 | 4.82 | 1.42 |
| 30 | 8.83 | 2.41 |

| Cyclosporin | | |
|---|---|---|
| 8 | 1.54 | 0.63 |
| 13 | 3.31 | 1.37 |
| 24 | 8.45 | 1.94 |
| 30 | 13.59 | 2.34 |

These results obtained in this standard pharmaceutical test procedure demonstrate that the compounds of this invention augment the neuroprotective effects of NMDA and AMPA inhibitors in inhibiting ischemia induced neurotoxicity.

The compounds of this invention can be formulated neat or with a pharmaceutical carrier to a mammal in need thereof. The pharmaceutical carrier may be solid or liquid. When formulated orally, it has been found that 0.01% Tween 80 in PHOSAL PG-50 (phospholipid concentrate with 1,2-propylene glycol, A. Nattermann & Cie. GmbH) provides an acceptable oral formulation.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally in the form of a conventional suppository. For administration by intranasal or intrabronchial inhalation or insufflation, rapamycin may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermeable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected intravenous daily dosages of active compound would be 0.1 µg/kg - 100 mg/kg, preferably between 0.001 - 25 mg/kg, and more preferably between 0.01 - 5 mg/kg. Projected daily oral dosages would be 0.005 - 50 mg/kg, preferably between 0.01 - 25 mg/kg, and more preferably between 0.05 - 10 mg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following examples illustrate the preparation of the compounds of this invention.

### Example 1

### Rapamycin

The preparation of rapamycin has been described in U.S. Patent 3,929,992.

### Example 2

### Rapamycin 1,3, Diels Alder adduct with Phenyltriazolinedione

Rapamycin (0.66g, 721 mmol) was dissolved in dichloromethane (10 ml) and cooled to 0 °C. To this was added, dropwise, a solution of phenyltriazolinedione (0.133 g, 758 mmol) in dichloromethane (10 ml). The solution was stirred overnight, TLC showed the reaction was not complete. Additional phenyltriazenedione (0.025g, 27 mmol) was added. The reaction was purified using HPLC (4.1x31cm, SiO₂) with ethyl acetate as eluant to provide the title compound as a solid. The solid was triturated with 30 ml of hexane and 1 ml of ethyl acetate filtered and air dried to give the title compound as a powder (0.383 g).
Anal Calc for C₅₉H₈₄N₄O₁₅: C, 65.05; H, 7.77; N, 5.14. Found: C, 65.39; H, 7.98; N, 4.92
IR (KBr, cm⁻¹) 3450, 1715
NMR (DMSO) δ 7.50 (m, 3H), 7.40 (m, 2H), 3.11 (s, 3H), 3.00 (s, 3H) 2.95 (s, 3H), 0.8(q,1H)
MS (-FAB) 1088 (M⁻)

### Example 3

### Rapamycin 42-ester with 4-[[4-(dimethylamino)phenyl]azo]benzenesulfonic acid

The preparation of the compound of Example 3 has been described in U.S. Patent 5,177,2030.

### Example 4

### Rapamycin 1,3-Diels Alder adduct with methyltriazolinedione

The title compound was prepared according to the method described in Example 2.

### Example 5

### Rapamycin-O-benzyl-27-oxime

The preparation of the compound of Example 5 has been described in U.S. Patent 5,023,264.

## Claims

1. Use of rapamycin, rapamycin 1,3-Diels Alder adduct with phenyltriazolinedione, rapamycin 42-ester with 4-[[4-(dimethylamino)phenyl]azo]-benzenesulfonic acid, rapamycin 1,3-Diels Alder adduct with methyltriazolinedione, or rapamycin-O-benzyl-27-oxime for the manufacture of a medicament for treating epilepsy or Huntington's Disease in a mammal.

2. Use as claimed in claim 1 in which the medicament is adapted for administration orally, parenterally, intravascularly, intranasally, intrabronchially, transdermally, or rectally.

3. Use as claimed in claim 1 or claim 2 wherein the compound is rapamycin.

4. Use according to any one of claims 1 to 3, in which the medicament further comprises an NMDA or AMPA antagonist.

5. Use according to Claim 4 in which the antagonist is:
[2-(8,9-Dioxo-2,6-diazabicyclo[5.2.0]-non-1(7)-en-2-yl)ethyl]phosphonic acid; D-amino-5-phosphonopentanoate (D-AP5), 4-phosphonomethyl-2-piperidine-carboxylic acid;
D, L-(E)-2-amino-4-methylphosphono-3-pentanoic acid;
5,7-dichlorokynurenate, trans-2-carboxy-5,7-dichloro-4-phenylaminocarbonylamino-1,2,3,4-tetrahydroquinoline; 5, 7-dinitroquinoxoline-2, 3-dione;
6-(1H-Imidazol-1-yl)-7-nitro-2,3(1H,4H)-quinoxalinedione hydrochloride;
1-(4-aminophenyl)-4-methyl-7,8-methylenedioxy-5H-2,3-benzodiazepine HCl;
6-nitro-7-sulphamobenzo(f)quinoxaline-2,3-dione (NBQX), 6-cyano-7-nitroquinoxaline-2,3-dione and 6,7-dinitroquinoxaline-2,3-dione.

6. Product containing rapamycin, rapamycin 1,3-Diels Alder adduct with phenyltriazolinedione, rapamycin 42-ester with 4-[[4-(dimethylamino)phenyl]azo]-benzenesulfonic acid, rapamycin 1,3-Diels Alder adduct with methyltriazolinedione, or rapamycin-O-benzyl-27-oxime and an NMDA or AMPA antagonist as a combined preparation for simultaneous, separate or sequential use for treating epilepsy or Huntington's Disease in a mammal.

7. A pharmaceutical composition comprising rapamycin, rapamycin 1,3-Diels Alder adduct with phenyltriazolinedione, rapamycin 42-ester with 4-[[4-(dimethylamino)phenyl]azo]-benzenesulfonic acid, rapamycin 1,3-Diels Alder adduct with methyltriazolinedione, or rapamycin-O-benzyl-27-oxime and a NMDA or AMPA antagonist and a pharmaceutically acceptable carrier.

## Patentansprüche

1. Verwendung von Rapamycin, Rapamycin-1,3-Diels-Alder-Addukt mit Phenyltriazolindion, Rapamycin-42-Ester mit 4-[[4-(Dimethylamino)-phenyl]-azo]-benzolsulfonsäure, Rapamycin-1,3-Diels-Alder-Addukt mit Methyltriazolindion oder Rapamycin-O-benzyl-27-oxim zur Herstellung eines Medikaments zur Behandlung von Epilepsie oder der Huntington'schen Erkrankung bei einem Säuger.

2. Verwendung nach Anspruch 1, wobei das Medikament für orale, parenterale, intravaskuläre, intranasale, intrabronchiale, transdermale oder rektale Verabreichung ausgelegt ist.

3. Verwendung nach Anspruch 1 oder 2, wobei die Verbindung Rapamycin ist.

4. Verwendung nach einem der Ansprüche 1 bis 3, wobei das Medikament weiters einen NMDA- oder AMPA-Antagonisten aufweist.

5. Verwendung nach Anspruch 4, wobei der Antagonist
[2-(8,9-Dioxo-2,6-diazabicyclo[5.2.0]-non-1(7)-en-2-yl)-ethyl]-phosphonsäure;
D-Amino-5-phosphonpentanoat (D-AP5), 4-Phosphonmethyl-2-piperidin-carbonsäure;
D,L-(E)-2-Amino-4-methylphosphon-3-pentansäure;
5,7-Dichlorkynurenat, Trans-2-carboxy-5,7-dichlor-4-phenylaminocarbonylamino-1,2,3,4-tetrahydrochinolin;
5,7-Dinitrochinoxolin-2,3-dion;
6-(1H-Imidazol-1-yl)-7-nitro-2,3(1H,4H)-chinoxalindion-hydrochlorid;
1-(4-Aminophenyl)-4-methyl-7,8-methylendioxy-5H-2,3-benzodiazepin-HCl;
6-Nitro-7-sulfamobenzo(f)-chinoxalin-2,3-dion (NBQX), 6-Cyano-7-nitrochinoxalin-2,3-dion und
6,7-Dinitrochinoxalin-2,3-dion
ist.

6. Produkt, enthaltend Rapamycin, Rapamycin-1,3-Diels-Alder-Addukt mit Phenyltriazolindion, Rapamycin-42-Ester mit 4-[(4-(Dimethylamino)-phenyl]-azo]-benzolsulfonsäure, Rapamycin-1,3-Diels-Alder-Addukt mit Methyltriazolindion oder Rapamycin-O-benzyl-27-oxim und einen NMDA- oder AMPA-Antagonisten als Kombinationspräparat zur gleichzeitigen, separaten oder sequentiellen Verwendung zur Behandlung von Epilepsie oder der Huntington'schen Erkrankung bei einem Säuger.

7. Pharmazeutische Zusammensetzung, umfassend Rapamycin, Rapamycin-1,3-Diels-Alder-Addukt mit Phenyltriazolindion, Rapamycin-42-Ester mit 4-[[4-(Dimethylamino)-phenyl]-azo]-benzolsulfonsäure, Rapamycin-1,3-Diels-Alder-Addukt mit Methyltriazolindion oder Rapamycin-O-benzyl-27-oxim und einen NMDA- oder AMPA-Antagonisten und einen pharmazeutisch akzeptablen Träger.

## Revendications

1. Utilisation de la rapamycine, de l'adduit de Diels Alder en 1,3 de la rapamycine avec de la phényltriazolinedione, du 42-ester de rapamycine avec l'acide 4-[[4-(diméthylamino)phényl]azo]benzène-sulfonique, de l'adduit de Diels Alder en 1,3 de la rapamycine avec de la méthyltriazolinedione, ou du rapamycine-O-benzyl-27-oxime pour la fabrication d'un médicament pour le traitement de l'épilepsie ou de la maladie de Huntington chez un mammifère.

2. Utilisation suivant la revendication 1, dans laquelle le médicament est adapté pour une administration orale, parentérale, intra-vasculaire, intranasale, intrabronchiale, percutanée ou rectale.

3. Utilisation suivant la revendication 1 ou la revendication 2, dans laquelle le composé est la rapamycine.

4. Utilisation suivant l'une quelconque des revendications 1 à 3, dans laquelle le médicament comprend en outre un antagoniste du NMDA ou de l'AMPA.

5. Utilisation suivant la revendication 4, dans laquelle l'antagoniste est :
l'acide [2-(8,9-dioxo-2,6-diazabicyclo[5.2.0]non-1(7)-én-2-yl)éthyl]phosphonique;
le D-amino-5-phosphonopentanoate (D-AP5), l'acide 4-phosphonométhyl-2-pipéridinecarboxyique;
l'acide D,L-(E)-2-amino-4-méthylphosphono-3-pentanoïque;
la 5,7-dichlorokynurénate, le trans-2-carboxy-5,7-dichloro-4-phénylaminocarbonylamino-1,2,3,4-tétrahydroquinoline;
la 5,7-dinitroquinoxoline-2,3-dione;
l'hydrochlorure de 6-(1H-imidazol-1-yl)-7-nitro-2,3-(1H,4H)-quinaxalinedione;
la 1-(4-aminophényl)-4-méthyl-7,8-méthylènedioxy-5H-2,3-benzodiazépine HCl;
la 6-nitro-7-sulfamobenzo(f)quinoxaline-2,3-dione (NBQX), la 6-cyano-7-nitroquinoxaline-2,3-dione et la 6,7-dinitroquinoxaline-2,3-dione.

6. Produit contenant de la rapamycine, de l'adduit de Diels Alder en 1,3 de la rapamycine avec de la phényltriazolinedione, du 42-ester de rapamycine avec l'acide 4-[[4-(diméthylamino)phényl]azo]benzène-sulfonique, de l'adduit de Diels Alder en 1,3 de la rapamycine avec de la méthyltriazolinedione, ou du rapamycine-O-benzyl-27-oxime et un antagoniste du NMDA ou de l'AMPA sous forme d'une préparation combinée pour une utilisation simultanée, distincte ou séquentielle pour le traitement de l'épilepsie ou de la maladie de Huntington chez un mammifère.

7. Composition pharmaceutique comprenant de la rapamycine, de l'adduit de Diels Aider en 1,3 de la rapamycine avec de la phényltriazolinedione, du 42-ester de rapamycine avec l'acide 4-[[4-(diméthylamino)-phényl]azo]benzènesulfonique, de l'adduit de Diels Alder en 1,3 de la rapamycine avec de la méthyltriazolinedione, ou du rapamycine-O-benzyl-27-oxime et un antagoniste du NMDA ou de l'AMPA et un vecteur pharmaceutiquement acceptable.
